# EUROPEAN PATENT SPECIFICATION

(11) **EP 2 401 920 B1**
(45) Date of publication and mention of the grant of the patent: **20.02.2013**
(21) Application number: 11172382.1
(22) Date of filing: 01.07.2011
(51) Int. Cl.: C07K 14/425, A21D 13/06, A21D 2/26, A23L 1/16

(54) **Method for the production of maize proteins and use of said proteins for the production of gluten-free bakery products and pasta**
Verfahren zur Herstellung von Maisproteinen und Verwendung besagter Proteine zur Herstellung von glutenfreien Backwaren und Pasta
Procédé pour la production de protéines de maïs et utilisation desdites protéines pour la production de produits de boulangerie et de pâtes sans gluten

(30) Priority: 02.07.2010 IT UD20100131
(43) Date of publication of application: 04.01.2012
(73) Proprietor: DR. SCHAER SPA, 39014 Postal (BZ) (IT)
(72) Inventor: Cerne, Virna Lucia, 34011 Duino-Aurisina (TS) (IT)
(74) Representative: Petraz, Gilberto Luigi

(56) References cited:
- WO-A1-2008/036646
- JP-A- 2004 242 647
- US-A- 3 535 305
- HOJILLA-EVANGELISTA M ET AL: "Optimizing extraction of zein and glutelin-rich fraction during sequential extraction processing of corn", CEREAL CHEMISTRY, AMERICAN ASSOCIATION OF CEREAL CHEMISTS. MINNEAPOLIS, US, vol. 80, no. 4, 1 January 1979 (1979-01-01), pages 481-484, XP009092386, ISSN: 0009-0352, DOI: DOI:10.1094/CCHEM.2003.80.4.481
- SCHOBER T J ET AL: "Improved viscoelastic zein-starch doughs for leavened gluten-free breads: Their rheology and microstructure", JOURNAL OF CEREAL SCIENCE, ACADEMIC PRESS LTD, XX, vol. 48, no. 3, 1 November 2008 (2008-11-01), pages 755-767, XP025611555, ISSN: 0733-5210, DOI: DOI:10.1016/J.JCS.2008.04.004 [retrieved on 2008-05-10]

## Description

### FIELD OF THE INVENTION

The present invention concerns a method for the production of maize proteins and the use of the proteins in formulas or recipes for the production of gluten-free and additive-free bakery products and pasta.

### BACKGROUND OF THE INVENTION

It is known that proteins in cereals can be classified into four fractions according to solubility (Osborne, 1970): albumins, globulins, prolamines and glutelins. The Osborne fractions deriving from different cereals are often identified with specific names. In maize, for example, the fraction of prolamines is called zein and the glutelins are called glutenins.

In wheat, the prolamines and glutelins are called gliadins and glutenins and form gluten. The fraction of glutenins is formed by a mixture of polymers with a high molecular weight (HMW) and a low molecular weight (LMW). Using SDS-PAGE electro-phoretic analysis, the HMW polymers have a molecular weight comprised between 80,000 Da and 160,000 Da, and the LMW polymers between 30,000 and 51,000 Da (Payne, P.I., Law, C.N., Mudd, E.E. 1980. Control of homoelogous group 1 chromosomes of the high-molecular-weight subunits of glutenin, a major protein of wheat endosperm. Theoretical and Applied Genetics 58, 113-120). The biggest polymers of glutenin are stabilized by inter-chain disulphide bonds.

In bakery, or oven, products, the common wheat flour (*Triticum aestivum*) is the main ingredient; it provides mass and structure to most bakery products, including bread, biscuits, cakes, pastry products and pasta. Wheat is the only one of the cereals whose flour has the ability to form the dough when it is mixed with water at room temperature (Lawton, 1992; Hui et al. 2006). The glass transition temperature (Tg) of a polymer marks the border between the amorphous glass state and the amorphous plastic state, a very rigid liquid characterized by great viscosity. Tg is one of the important parameters for defining the properties of proteins and is very sensitive to modifications and the presence of plasticizers. Water is one of the best plasticizers for gluten and causes a drastic reduction in its glass transition temperature. To have good visco-elastic properties, proteins must be above their glass transition temperature and this must be kept in consideration when other cereals are to be studied which do not form visco-elastic doughs at room temperature like wheat glutin (Singh and MacRitchie, 2001). The factors that contribute to good quality in the leavened products are correlated to the amino-acid composition, the structure and the polymer nature of the proteins in the dough. The gluten in wheat dough is able to retain the gas produced during fermentation. The dough needs a balance between elasticity and extensibility so as to maximize its performance when baked in the oven. Gluten polymers with a higher molecular weight help to form a more elastic structure in bread (Atwell, 2001). The flour is called "strong" when it has a higher concentration of gluten. Flour like this absorbs a large quantity of water to make an elastic dough and has high ability to retain carbon dioxide, creating a good protein reticulation (Stringher, 2006).

Prolamines are present not only in wheat, but also in other cereals like oats, barley, rye, emmer, spelt and triticum wheat.

Coeliac disease, permanent intolerance to gluten, is one of the most widespread food intolerances that affect the small intestine. It affects about one person in every 100-200, mostly in Europe, North and South America and Australia.

In the person who is genetically susceptible, both adult and child, the ingestion of foodstuffs containing gluten, even only in small quantities, causes an immune reaction in the small intestine, causing a chronic inflammation which in turn causes the villi of the intestine to disappear, accompanied by symptoms that vary from case to case. The flattened absorbent surface reduces or prevents nutritional substances from being absorbed, such as proteins, fats, carbohydrates, vitamins and mineral salts, causing malnutrition and decompensation.

A rigorously gluten-free diet is still today the only effective treatment against gluten-intolerance. Those affected by coeliac disease can cover their need for nutrients with food that is naturally gluten-free like meat, fish, vegetables, eggs, milk, potatoes or rice, and take the missing nutrients from gluten-free dietary products available on the market.

Bakery products and pasta are foods that provide the greatest contribution of carbohydrates in our diet, and are very important nutritionally for a balanced diet. Obviously, improving the quality of gluten-free products is the main challenge to increase consumption and promote the spread of a more balanced diet among coeliac sufferers.

In many cases the sensory properties and texture are poor compared with gluten-based bakery products. The absence of gluten in doughs means that unworkable liquid-viscous products are obtained, and the final product (bread) is without structure, very distant in organoleptic quality from traditional wheat products.

In order to obtain satisfactory properties of gluten-free bread, usually a lot of ingredients and additives are required.

Among the vegetable proteins different from gluten, the most interesting are zein and maize glutelins. Zein is the main storage protein of maize and accounts for about 44%-79% of the endosperm, according to the variety of maize and the separation methods used (Landry et al., 2000). Zein is localized in protein bodies (Shimamoto et al., 1983) which have a diameter of about 1-4 micrometers (Paiva et al., 1991). Analysis of the amino-acid composition indicates a large quantity of glutammic acid (21-26%), leucine (20%), proline (10%), alanine (10%), and a lack of lysine and tryptophan (Shukla and Cheryan, 2001; Hui et al., 2006). The high percentage of non-polar acid residues and the exceptional lack of ionic groups are responsible for the highly hydrophobic nature of zein (Hiu et al, 2006).

In accordance with the Landry-Moureaux (1981) system, zein is a prolamine characterized by solubility in alcohol. The classic procedure to isolate and quantify it is to extract with an aqueous solution of ethyl- or isopropyl-alcohol in the absence and then in the presence of a reducing agent which makes the extraction quicker. Zein is a heterogeneous mixture of disulphide aggregates which vary in the molecular weight and the degree of solubility (Meija et al., 2007). α-zein is the most abundant and is about 70% of the total protein fraction (Thompson and Larkins, 1989).

Maize glutelins (CG) are proteins with a high molecular weight and represent 40% of the proteins of the endosperm. Glutelins are positively correlated with the lysine content of the endosperm (Yau et al., 2002).

Although traditionally non-prolamine proteins have been divided into albumins, globulin and glutelins, the lines of division are very difficult to trace and strictly depend on the separation methods used (Watson and Ramstad, 1999). The extraction of glutelin is not possible without a contamination of the most representative fractions of zein.

Corn Gluten Meal (CGM) is one of the sub-products of grinding maize. It mainly consists of proteins (about 69%). At present large quantities of CGM are available as an economic source of raw material for zootechnics. Zein is the protein fraction most common in CGM (as much as 47%).

Commercial zein consists only of α-zein (Wilson, 1988). It is not possible to use zein in its natural, non-purified form because it is contaminated by other substances. In particular, it contains pigments like xanthophylls and carotenoids which, if used in bakery products, give a strong and unpleasant color, smell and taste.

Zein is produced by different industries. Methods and systems for the industrial extraction of zein are described in a large number of patents and patent applications. Usually solutions with 70% ethanol at 40-50°C are used for various times, followed by whitening processes because, since it derives from yellow maize flour, the color of the zein as extracted is affected by this.

Purified zein is colorless and odorless. The purification process is complex and costly. For example the patents US5367055 and US534292 concern the use of acetone for refining zein after extraction. The patent US-B-616031 and the application US-A-2005/0049400 concern the possibility of industrial extractions of the protein, but use a very complex plant and a large quantity of solvent.

For the moment, zein has few applications and is mainly used in the pharmaceutical industry for producing edible films for covering drugs (patent US3116206, 1963; Takahashi et al., 2002). Many studies have been carried out to improve industrial zein quantities with reduced costs (Shukla et al., 2000; Cheryan, 2000, 2006 a,b; Parris and Dikey, 2001; Lawton, 2006; Xu et al., 2007; Sellino and Woods, 2008). The development of technologies that will provide a low-cost way to isolate zein will allow it to enter into new markets (Sellino and Woods, 2008).

At this moment in time commercial zein is very costly and cannot be used to replace gluten in gluten-free food products.

Glutelins are not taken into consideration in industrial extractions even though some patents talk about a "glutelin-rich fraction" in protein residues of maize after the extraction of the zein. The article by Mila P. Hojilla-Evangelista and Lawrence A. Johnson, 2003, "Optimized extraction of zein and glutelin rich fraction during sequential extraction processing of corn" - Cereal Chemistry 80(4):481-484, focuses on evaluation methods for increasing the recovery of zein and GRF (glutelin-rich fractions) from maize. These methods use different extraction passes for zein and the GRF and apply a filtration system with a membrane in order to recover the proteins and to reduce the costs of evaporation of the solvent.

On a chemical-physical level, the secondary structure of zein is approximately 50% α-helical and about15% β-helical (Wu et al., 1971). Unlike wheat gluten, maize zein, when hydrated, does not form visco-elastic dough, even if this can be achieved at higher temperatures (Bugusu et al., 2001; Lawton, 1992).

The changes in the secondary structure of zein are similar to those which are found in gluten during the formation of the dough. The stability of the polymers in the visco-elastic system of gluten is correlated to the extended network of β -layer structures stabilized by the glutenins. Instead, the extended alignments of β -layer structures that occur during the formation of the visco-elastic polymer zein do not remain stable, and rapidly relax due to the lack of subunits with a higher molecular weight (HMW) (Mejia et al., 2007).

The patent application WO-A-2008/036646 shows the differences in the profiles in a mixograph between a wheat dough at 25°C and doughs with zein (10%)-starch and at 35°C. In the zein-starch dough a lower development time was observed, and a greater resistance (high peak on the mixograph) with respect to the wheat dough.

If the stress is removed, or at room temperature, both resistance to stress and the visco-elasticity of the zein system are lost.

Meija et al (2007) report that the temperature and shearing stress are necessary not only to keep the visco-elastic nature of the zein polymer, but also to keep the β -layer structure.

MacRitchie, Carson B.A., Sroab B.S. in a poster of 2004 entitled "Can quality aerated products be made from non-wheat cereals?" report on some analyses made on zein-starch doughs which show the visco-elastic properties of such doughs at 40°C. However, the poor properties of gas retention, which reflect on the absence of capacity of tension-hardening, are due to the lack of large molecules such as for example the glutelins.

In gluten-free doughs, the enzymes can help the formation of crossed bonds between proteins. The addition of transglutaminase (Tgase) slightly influences the formulations with oat flour, sorghum and teff, but on the contrary has negative effects on the pseudo-plastic behavior of maize flour doughs (Renzetti et al., 2008). There are no studies on the use of Tagse in gluten-free doughs with maize glutelin. Maize glutelins have 5.4% lysine (Sodek and Wilson, 1971) and at higher concentrations may probably be used as targets for Tgase activity.

Glucose oxydase (GOx) is a natural oxidizing agent. In the presence of oxygen this enzyme catalyzes the reduction reaction of β-D-glucose into D-glucono-δ-lactone and the formation of hydrogen peroxide (H₂O₂) which oxidizes the SH-group in disulphide bridges (-S-S-) of protein structures.

Other additives used to improve the characteristics of flour during kneading are reduction and oxidation agents (redox). Their main effect is on the SH/SH systems of the flour. For economic reasons L-cysteine is the most commonly used reducing agent. L-cysteine acts on the disulphide bridges (SS) in the dough and breaks them up, reducing them to sulfhydryl groups (SH). It reduces the development of the bread and its stability and helps in the final packaging of the dough without breaking its structure. L-cysteine is used in quantities comprised between 30 and 70 mg per kilo of flour. Values above this make the dough sticky and difficult to knead (Fitchett and Frazier, 1986). Oxidizing agents, on the contrary, are often used both as whiteners, for their oxidizing effect on the pigments, and also to improve the rheological properties of the dough and its ability to retain gas. The use of oxidants is more tolerated in the US and has more restrictive conditions in Europe, where only ascorbic acid is allowed.

Ascorbic acid (AA) can help to retain gas thanks to its effect on the protein structure of the dough. During mixing when there is oxygen and ascorbic oxydase enzyme present, the AA is converted into dehydroascorbic acid (DHA). The oxidation reaction of the AA in the dough is complex but probably helps the oxidation of the SH groups of the proteins and the formation of the SS bridges between the protein structures. The final effect is to obtain an elastic and stable protein structure able to retain the gases so as to be able to develop a bread with a good internal alveolar structure. The legal limit for the use of ascorbic acid is 0.02% with respect to the flour (Quaglia, 1984).

The combination of the two effects of reduction and oxidation of the redox agents in the right proportions is able to create easily workable doughs with good rheological properties.

In one formulation of bread based on rice (also containing methyl cellulose), the presence of the GOx enzyme improves both the rheological properties of the dough, the specific volume and also the texture of the bread (Gujral et al., 2004). GOx is used at 0.01-0.02%, maximum 0.03% of the weight of the flour. Larger quantities of GOx create excess H₂O₂, a reduction in the viscosity of the watersoluble fraction and hence a worsening of the conditions necessary for forming a dough.

Gluten-free bread and bakery products with commercial zein are already known.

Application WO-A-2008/036646 concerns the preparation of a mixture for bread comprising starch, zein and a co-protein, such as casein or elastin. The co-protein stabilizes the β-layer structures of the zein, which facilitates the retention of CO₂ for leavening. The addition of co-protein contributes to maintain the resistance to stress and visco-elasticity of the zein-starch dough for longer periods and at room temperature. It must be added that this is a liquid dough and that the commercial zein used here confers an unpleasant odor and a strong yellow color to the bread. Moreover, the casein is not distributed homogeneously in the dough and creates darker lumps.

Application US-A-2008/0038434 to replace gluten in food products uses various polymers (additives) as well as zein. The polymers inserted into the formula must have the function of retaining gas (gas retaining agent) and also of catalyzing the polymerization (setting agent). For example hydrophilic polymers, hydrocolloids and pre-gelatinized starches have the function of retaining the gases. On the contrary, polylactic acid, polyvynil alcohol, maize zein and polycaprolactone help in polymerization. Different formulas have been made for gluten-free products with these polymers, but there is no description of the fact that zein is useful in such doughs. Indeed, the effect of the zein may be hidden by the other additives such as for example cellulose and hydroxypropyl methyl cellulose (HPMC). The application US-A-2008/0038434 provides to combine several polymers and not to use only zein as a substitute for gluten.

Bugusu, B.A., Campanella, O., Hamaker, B.R., 2001 "Improvement of sorghum-wheat composite dough rheological properties and breadmaking quality through zein addition", 2001. Cereal Chemistry 78(1), 31-35 reports that the addition of α-zein (<2% of the total weight of the flour) at 35°C improves the leavening of the dough and the rheological characteristics of the bread made with wheat flour and sorghum-wheat (20-80). This study in any case provides to use wheat flour and therefore is not suitable for coeliacs.

Oom A., Petterson A., Taylor J.R.N., Stading M., 2008 "Rheological properties of kafirin and zein prolamins." Journal of cereal Science, 47(1), 109-116 compare kafirin and zein with wheat gluten. Despite the differences in molecular weight and their hydrophobic nature, the resins consisting of kafirin and zein in certain conditions have similar rheological properties of extensibility with respect to wheat gluten. There is no reference to how to make a gluten-free bread dough in such conditions.

Schober T.J., Bean S.C., Boyle D.L. and Park S.H., 2008 "Improved viscoelastic zein-starch doughs for leavened gluten-free breads: their rheology and microstructure", J. Cereal Science. 48(3):755-767, like the application US-A-2008/0038434, rely on the importance of adding hydrocolloids to the dough with zein. They describe a good gluten-free bread (for example soft pretzels) made with yellow zein, wheat starches and HPMC (hydroxypropyl methyl cellulose). The article also describes tests carried out with zein of different grain size and concludes that the small particles of the zein probably cause the weakness of the dough. When the dough with zein is mixed at lower temperatures than the glass transition temperature of such proteins, the protein filament loses resistance to mechanical extension and the dough becomes stronger and less extendible. The yellow zein used for these tests makes the bread too colored and is too different from the typical wheat bread.

The quality of all the breads described in the patents and articles discussed above is very poor. A real substitution of wheat gluten by zein does not provide workable doughs and increases the hardness of the bread after a few hours. Furthermore, yellow zein contains colored pigments which give an accentuated yellow color to the bakery products, and also a pungent smell and an unpleasant taste.

One purpose of the present invention is to produce a maize protein than can replace wheat gluten in bakery, or oven, products and in pasta.

Another purpose of the present invention is that the protein obtained has a behavior and function similar to wheat gluten in doughs. The main characteristic required for the protein according to the present invention is the ability to reticulate a dough with the right contribution of plasticizers (for example water), the right temperature (higher than the glass transition temperature of the protein) and the right quantity of mechanical energy transferred during kneading. The dough thus obtained must have rheological characteristics similar to those of wheat, such as elasticity, strength, viscosity and stability during kneading. All this, while avoiding the use of thickeners and additives which are present in most products on the market and in the patents and scientific articles cited above.

A further purpose is that the protein produced and used is able to confer taste, odors and colors to the finished product similar to traditional products with wheat flour.

Another purpose is that the protein must have a natural origin, vegetable, suitable for coeliacs and available in large quantities.

Furthermore, the protein must be usable economically in gluten-free foods and for this reason one of the purposes of the present invention is that the extraction and preparation process must be economical and must give all the main protein fractions.

Another purpose is that the finished product prepared according to the methods using protein fractions obtained as described must be similar to traditional bakery products from wheat flour, such as taste, color, odor and structure (specific weight, alveolar structure, hardness).

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

The above purposes are obtained from the inventive concept according to the present invention and proposed by Applicant, which is to use maize glutelin as well as zein, deriving from white maize in a leavened bakery, or oven, product or other gluten-free type.

A method for the production of maize proteins according to the present invention provides an extraction step to extract, from white maize and/or its by-products, such as for example, but not only, white maize flour, CGM (Corn Gluten Meal) or other, protein fractions comprising zein and glutelin, using an aqueous solution based on an alcohol or mixtures of alcohol, in the absence of reducing or purifying agents. That is, the present invention does not provide in the extraction step either to use a reducing or purifying agent as a whitening agent.

The absence of a reducing and whitening agent makes the extraction method according to the present invention compatible with the extraction, together with zein, of glutelins too. This because the glutelins, in the presence of reducing or whitening agents, would be degraded and would be unusable.

One form of embodiment of the present invention provides to use, in the extraction, an aqueous solution based on isopropanol.

According to some forms of embodiment of the present invention, one or more of the protein fractions zeins and glutelins are extracted.

According to some forms of embodiment of the present invention, the extraction step provides to use a buffer to control the extraction pH, for example a phosphate buffer.

The buffer serves to maintain an optimum extraction pH in a range comprised between about 5 and about 8, preferably between about 6.5 and about 8, for example about 7.6. The optimum extraction pH values have been experimentally identified by Applicant as values which advantageously determine a more favorable environment for the extraction of maize proteins desired, that is, zein and glutelin.

In some forms of embodiment, the concentration of isopropanol used goes from about 40% to about 90% (molar concentration). Advantageous variants, which allow to save on the chemical solvents used, provide a concentration of isopropanol of about 50%.

In some forms of embodiment, the extraction step comprises mixing white maize flour with the aqueous solution of alcohol and, possibly, the phosphate buffer, then heating and stirring.

In some forms of embodiment of the present invention, the extraction takes place in a heating container which can be closed.

In some forms of embodiment, the heating takes place at a temperature of about 35°C to about 60°C. An advantageous variant provides to perform the heating at about 50°C.

In some forms of embodiment of the present invention, as soon as the mixture of white maize flour and aqueous solution of alcohol has reached the desired temperature, the stirring and mixing is started. In some variants of the present invention the stirring lasts from about 10 minutes to about 100 minutes. An advantageous variant provides stirring to last about 20 minutes.

In some forms of embodiment of the present invention, the extraction step is repeated a plurality of times. In some variants, the extraction step is repeated from 2 to 6 times. In some forms of embodiment, a repetition of from 2 to 4 times, for example 2 times, of the extraction step provides an optimum result to save time and solvent. The repetition of the extraction step allows to obtain an optimum quantity of proteins from white maize flour extracted, that is, zein and glutelin, without having recourse to reducing agents.

In other forms of embodiment, a separation step is provided, suitable to remove from the protein extract those components that are not soluble in the aqueous solution of alcohol.

In some forms of embodiment of the present invention, the excess alcohol and water in the extract is removed by evaporation. In some variants, the thermal exposure time of the extract to evaporation is very short, advantageously between 2 and 7 seconds. Alternatively, it is possible to carry out filtering, using a pressurized tubular plant and an ultra-filter module which separates the particles of less than 10 KDa. Other forms of embodiment of the present invention provide to pulverize the extract.

The present invention also concerns proteins from white maize that can be obtained using a method as described above.

Another feature of the present invention concerns a formula or recipe for the preparation of a food comprising proteins from white maize extracted using a method as described above.

In some forms of embodiment, the percentage of protein fractions extracted with the present invention with respect to the starch to be inserted in the dough is comprised between about 5% and about 30%.

In some forms of embodiment, the formula comprises the proteins extracted according to the present invention at 20%.

In some forms of embodiment, the formula is used to make a dough for bakery products, leavened or not, gluten-free, such as bread, biscuits, cakes, pasta or other. The bakery products and pasta are comparable in quality with traditional bakery products with gluten.

In some forms of embodiment, a dough for bakery products comprises the above formula, starches and gluten-free flours, the enzyme glucose oxydase (GOx) or transglutaminase, or redox agents such as ascorbic acid and L-cysteine, yeast, oil, such as vegetable oil, for example sunflower oil, palm oil or other, sugar, such as dextrose, fructose or other, salt and water.

In some forms of embodiment of the dough, the percentage in weight of the dry ingredients of glucose oxydase in the above formula is comprised between about 0.005% and about 0.05%.

In some forms of embodiment of the dough, the percentage in weight of the dry ingredients of ascorbic acid in the above formula is comprised between about 0.005% and 0.02%.

In some forms of embodiment of the dough, the percentage in weight of the dry ingredients of L-cysteine in the above formula is comprised between about 0.03% and about 0.07%.

In some forms of embodiment of the dough, the percentage in weight of yeast in the above formula is comprised between about 3% and about 7%, preferably between 4% and 6%.

In some forms of embodiment of the dough, the percentage in weight of oil in the above formula is comprised between about 4% and about 8%, preferably between 5% and 7%.

In some forms of embodiment of the dough, the percentage in weight of sugar in the above formula is comprised between about 0.5% and about 3%, preferably between 1% and 2.5%.

In some forms of embodiment of the dough, the percentage in weight of salt in the above formula is comprised between about 0.5% and about 1.5%.

In some forms of embodiment of the dough, the percentage in weight of water in the above formula is comprised between about 30% and about 90%, preferably between 50% and 70%.

In some forms of embodiment for the preparation of the dough according to the present invention, kneading is carried out at a temperature comprised between about 35°C and about 60°C.

In some forms of embodiment for the preparation of an bakery product using the dough according to the present invention, it is advantageous to prepare and keep the dough, both during the kneading step and also during the subsequent and leavening steps, until it goes into the oven, at a temperature comprised between about 35°C and about 60°C. With this range of temperatures between about 35°C and about 60°C the dough is kept above the glass transition temperature of the maize zein and glutelin and therefore they are in plastic form and with the contribution of mechanical kneading energy they plasticize and reticulate effectively, creating an optimum dough.

In some forms of embodiment, the kneading time goes from about 2 minutes to about 90 minutes.

In other forms of embodiment the formula is used to make pasta.

In some forms of embodiment, the formula of hydrated dough for pasta provides maize flour and/or rice flour in percentages from about 10% to about 90%, preferably from 70% to 90%, in weight and proteins produced according to the present invention from about 3% to about 7%. The gluten-free pasta obtained is comparable to traditional wheat pasta. The present invention also concerns the pasta comprising a formula as described above.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 shows a comparison of the chromatogram RP-HPLC of the protein fractions obtained from commercial zein and from white maize flour after extraction with 50% aqueous isopropanol at 50°C following the method of the present invention;
- fig. 2 shows the temperature profile in the Mixolab; curve A is a dough of 10% zein + wheat starch with an 86.4% absorption of water, and curve B a dough of 12% gluten + wheat starch with an absorption of 74.9%. The first minutes for stabilizing the dough are in relation to the optimum temperatures for the proteins used in the two doughs (wheat zein and gluten);
- fig. 3 shows the results obtained with the Kieffer Rig on the extensibility of the doughs with different percentages of zein and glucose oxydase and wheat starch kneaded at 40°C;
- fig. 4 shows an example of bread obtained following the recipe in example 2 of the present invention;
- fig. 5 shows the comparison of an electrophoretic SDS-PAGE analysis between maize proteins extracted according to the present invention in band 2, and maize proteins extracted in reducing conditions in band 3. Band 1 shows the standards of molecular weights.

### DETAILED DESCRIPTION OF A PREFERENTIAL FORM OF EMBODIMENT

The present invention follows a method for the production of gluten-free bread using the proteins from white maize as the only substitutes for wheat gluten, without additives or thickeners. Moreover, the method according to the present invention is advantageously economical for extracting proteins from white maize and/or its derivatives, such as for example flour, CGM (Corn Gluten meal) or others. The white maize proteins extracted maintain their functionality since in the extraction process reducing agents are not used, as is done instead for the maize proteins or zeins available on the market.

White maize does not contain colored pigments and for this reason the extraction process from white maize flour and its by-products present in this invention does not need further purification or whitening processes. Furthermore, the present invention extracts the main protein fractions of the maize, that is, the zeins and glutelins, since both perform an important functional role in the elasticity and strength of the dough, whereas in the extraction of commercial zein the glutelins are not considered and therefore are not extracted.

In the extraction of proteins from white maize an aqueous solution of alcohol is used, in this case it is advantageous to use aqueous isopropanol, and possibly a buffer to control the pH, for example a phosphate buffer. The buffer serves to keep an optimum extraction pH in a range comprised between about 5 and about 8, preferably between about 6.5 and about 8, for example about 7.6.

The solution obtained after extraction may be filtered several times, dried and then preserved in the form of dry powder. The product obtained contains zeins and glutelins at 65-90% of its weight. It should be noted that the final product may contain impurities which may include proteins other than zein and glutelin, such as albumins, globins and starch residues.

All the residual diluted isopropanol is preferably collected and re-concentrated so that it can be re-used. The system for collecting the isopropanol increases the efficiency of the system. The concentration of isopropanol used in the system goes from 40% to 90%, but the best extraction for saving chemical solvents is obtained at a concentration of 50%. The mixing is carried out in an explosion-proof container with double heating thickness which can possibly be closed. The extraction can be carried out approximately from 35°C to 60°C, but the best result is obtained at 50°C. As soon as the mixture of white maize flour and isopropanol has reached 50°C, the mixing mechanism (KPG-stirrer) begins, and lasts from 10 to 100 minutes, preferably 20 minutes. The operation can be repeated 2-6 times, but the best result for saving time and solvent is obtained by repeating the extraction 2-4 times.

To remove from the protein extract those components that are not soluble in aqueous isopropanol, a centrifuge or filtering process is used, preferably using a high-pressure, continuous-flow tubular filtering system. The Begerow filters used by the system go from 0.6 µm to 30 µm, preferably 2.5 µm. Typically the insoluble compounds are starch, cellulose and other proteins other than zein and maize glutelins.

To remove the excess isopropanol and water in the extract an evaporator is used, preferably a thin layer evaporator, and with an evaporation area of 1 m² at a temperature from 50°C to 100°C, preferably 70°C. The thermal exposure times of the mixture in the evaporator are very short, between 2 and 7 seconds, preferably 4 seconds, to avoid protein alterations. Alternatively, to remove the excess solvent from the extract a pump is used, connected to a module for ultra-filtering so that the concentration of alcohol does not decrease (and the proteins do not precipitate quickly), and even particles smaller than 10 KDa are separated from the rest (albumins, globulins, salts). In some forms of embodiment, the filter system is used with a flow comprised between 30 Kg/h to 90 Kg/h, preferably 60 Kg/h. To reduce the extract to powder a lyophilizer is used, preferably a spray dryer.

The white maize proteins extracted are analyzed with RP-HPLC (Reverse phase - high performance liquid chromography).

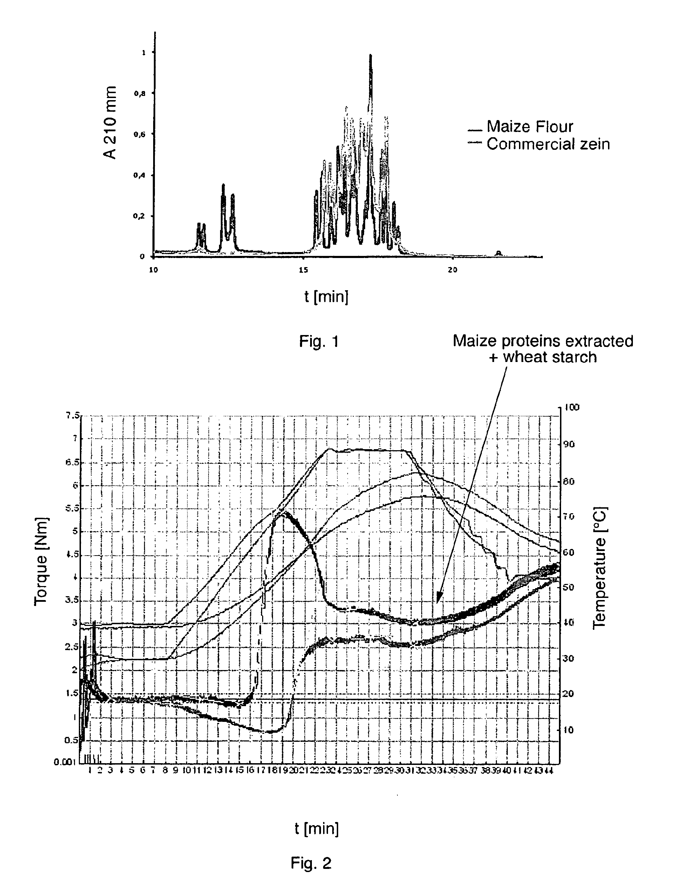

Fig. 1 shows a comparison of the chromatogram RP-HPLC of the protein fractions obtained from commercial zein and from white maize flour after extraction with 50% aqueous isopropanol at 50°C following the method described in the present invention. The fraction of the α-zeins extracted according to the method described is less representative (64.5% of the total proteins) with respect to the corresponding protein fraction of the zein present on the market (89.2% of total proteins). Moreover, the protein fractions of β-and γ-zeins are present in the proteins extracted according to the method described (between 11 and 13 min); on the contrary, these fractions are not present in commercial zein. The presence of the protein fractions β-and γ-zeins as well as the α-zeins in the protein extract obtained following the procedure described underlines the fact that they were not pre-treated with reducing agents which could influence the functionality of the proteins themselves. The β- and γ-zein fractions extracted according to this procedure have the advantage that they contain the amino-acid cysteine in higher quantities than α-zein, and consequently help the formation of disulphide bridges during kneading in particular circumstances, as described hereafter.

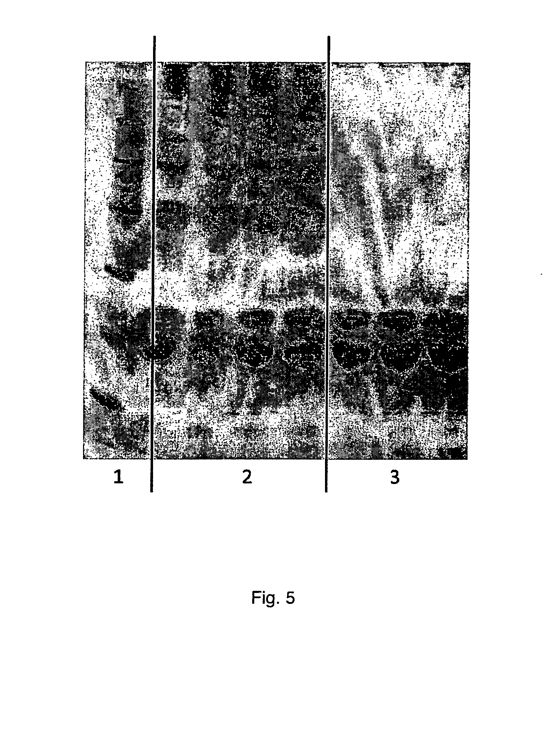

Fig. 5 shows the comparison of an electro-phoretic SDS-PAGE analysis between maize proteins extracted according to the present invention in band 2, and maize proteins extracted in reducing conditions in band 3. From the analysis of fig. 5 it can be seen clearly that the proteins extracted in reducing conditions, band 3, have lost the protein fractions with higher molecular weight, which instead are present in the proteins extracted in non-reducing conditions according to the present invention, in band 2.

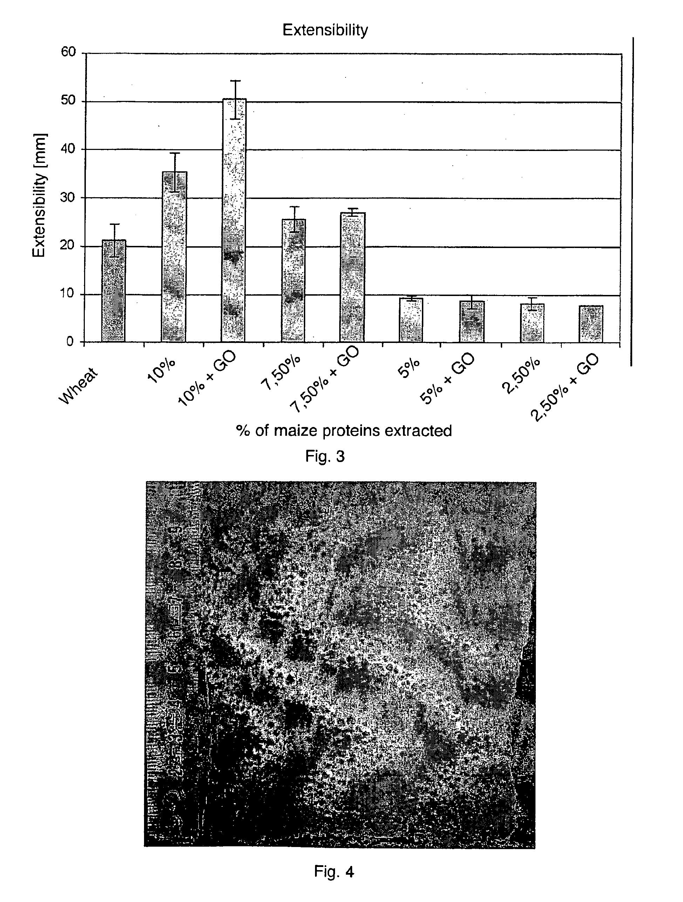

The quantities of protein fractions to be used in the dough were calculated according to analyses on the temperature profiles and stability, carried out using a Mixolab, and analyses on the extensibility and strength of the dough carried out using the Kieffer Rig (Texture Analyzer, model TAXT2i) (fig. 3). Preferably, the percentage of the protein fraction on the quantity of starch to be inserted in the dough goes from 5% to 30%, preferably 10%. In the temperature profile present in fig. 2, the stability of the dough is represented in the first heating step, before the thermal ramp. The dough was worked at an optimum temperature from 35°C to 60°C, preferably 40°C. The optimum kneading time for transferring a proper quantity of mechanical energy and obtaining a plastic and well-reticulated dough goes from 2 to 90 minutes, preferably from 2 to 60 minutes, for example 8 minutes. This allowed to obtain a strong and extendible dough. The protein fractions were modified physically and enzymatically so as to be able to increase their functionality in the dough. The enzyme glucose oxydase (GOx) is an enzyme that in the presence of oxygen catalyzes β-D-glucose into D-glucono-δ-lactone, and during this reaction forms H₂O₂ which in turn oxidizes the SH groups of the protein structures in groups S-S-. The enzyme glucose oxydase is used in percentages of 0.01-0.03% on the dry base of the dough, since these percentages create a protein reticulation which gives a good result on the extensibility of the dough. The extensibility of the dough measured with the Kieffer Rig (fig. 3) is preferably around 50 g after 30 minutes rest at a temperature from 35°C to 60°C, preferably 40°C.

The present invention also concerns the formula for a recipe for gluten-free bread with the proteins extracted according to the present invention. The bread produced according to the present invention has properties comparable to those of wheat bread. The present invention does not use additives which retain gas, or regulating agents such as thickeners such as for example hydrophilic polymers (hydrocolloids) and celluloses or hydroxypropylmethyl cellulose. The proteins obtained by the procedure described in the present invention are useful as the sole substitutes of gluten proteins present in wheat flour.

The percentage of protein fractions with highest molecular weight like the glutelins used in the dough according to the present invention varies from 2 to 100% of the total proteins used in the dough, preferably 50%.

The temperature of the dough in the food-mixer goes from 35°C to 60°C, preferably 40°C to maintain a better environment for the functionality of the white maize proteins according to the present invention. In order to keep the temperature during kneading, the temperature of the water added in the recipe goes from 35°C to 60°C, preferably 40°C. It is important that the temperature of the dough does not go below 35°C during the various passages until it reaches the oven, since this would compromise the structure of the disulphide protein bridges of the protein fractions extracted. The elastic structure of the protein fraction from maize remain such only if its ideal temperature is maintained in the dough. The mold too is kept at 35°C to 60°C, preferably 45°C. The passage from the food-mixer to the mold occurs at an optimal temperature from 35°C to 60°C, preferably 40°C. The leavening temperature used is calculated taking into consideration both the optimum temperature of the white maize proteins extracted and also the temperature range in which the yeast is active. The leavening step is effected in a leavening cell which keeps the temperature from 35°C to 50°C, preferably 40°C, and a humidity from 60% to 100%, preferably 95%. The leavening time is calculated according to tests on the activity of the yeast with cylinders and was fixed at from 10 minutes to 60 minutes, preferably 35 minutes. The passage from the leavening cell to the oven occurs at an optimum temperature from 35°C to 60°C, preferably 40°C. All the steps for preparing the dough, leavening and subsequent steps, until it is put in the oven, are carried out at a constant temperature in a range comprised between 35°C and 60°C. Cooking is done in the oven at an optimum temperature from 120°C to 250°C, preferably around 180°C for a time that depends on the size of the dough.

The hardness of the bread after one day and after seven days after cooking was analyzed using the Texture Analyzer (model TAXT2i). Preferably the hardness of the bread according to the present invention after 1 day is between 200 g and 300 g and after 7 days is between 400 g and 600 g. Preferably the bread obtained according to the present invention has a specific volume of minimum 4.5 cm³/g.

The present invention also concerns a method to produce gluten-free pasta with the proteins extracted using the method according to the present invention. The mix for the production of pasta consists of maize flour and/or rice flour and/or other flours and starches in varying percentages and a quantity of proteins according to the present invention from 2% to 30%, preferably from 4% to 20%, for example 15% of the weight of the hydrated dough. The enzyme transglutaminase, which catalyzes the acyltransferase reactions by introducing co-valent protein bonds, is used in percentages of 0.1 - 1.5% on the dry base of the dough since these percentages create a protein reticulation that provides a good result on the extensibility of the dough. The humidity of the dough must be comprised between 20% and 50%, preferably between 25% and 45%, for example 30%. The gluten-free pasta was extruded with extrusion temperatures from 20°C to 200°C using different temperature gradients. The pasta was dried either at room temperature or with a dryer. With this procedure, the gluten-free pasta obtained reaches similar levels of hardness, chewiness, palatability and rubberiness as traditional wheat pasta.

### EXAMPLES OF SOME FORMS OF EMBODIMENT OF THE PRESENT INVENTION

### EXAMPLE 1: extraction

The white maize flour was extracted repeating the extraction step four times (proportion 1:8) with a quantity of aqueous solution of isopropanol of 50% containing a phosphate buffer (pH =7.6).

The extraction solution was prepared as follows:
1. Solution KH₂NaPO₄: 1.14 g dissolved in 1 L (890g) of aqueous isopropanol 50% (v/v) (c = 0.009 mol/L)
2. Solution Na₂HPO₄*H₂O: 1.49 g dissolved in 1 L (890 g) of aqueous isopropanol 50% (v/v) (c = 0.009 mol/L)
3. The Na₂HPO₄*H₂O solution is taken to pH = 7.6 with the solution KH₂NaPO₄.

The albumins and globulins are not pre-extracted, but are dissolved with the zein during the alcohol extraction.

400L of aqueous solution of isopropanol at 50% containing a phosphate buffer are heated to 50°C in a container with a double heating thickness with a capacity of 500 liters and able to be closed; afterward, the flour is added to the solution. As soon as the mixture has reached the required 50°C, the mixing mechanism (KPG stirrer) is set for a time of 20 minutes. Subsequently the solution is cooled to room temperature. Then the proteins in solution are separated from the starch using a filter system under pressure and continuous flow with Begerow filters that go from 0.6 µm to 30 µm, preferably 2.5 µm. The extraction is repeated two more times on the residue of the white maize flour with the solution of isopropanol at 50% containing a phosphate buffer (proportion 1:4).

The extract is concentrated in a thin layer evaporator (evaporation area 1 m²) at 70°C for 3-5 seconds so that 75% of the solvent is removed (including the isopropanol) with the lowest thermal exposure times. Alternatively, to remove the excess solvent from the extract, everything is passed through an ultra-filtering system connected to a pump. The ultra-filtering system proportionally reduces the quantity of alcohol and eliminates the particles smaller than 10 KDa. The waste is collected in a container and weighed continuously to understand how quickly the concentration step is proceeding. Preferably, the flow used goes from 30 Kg/h to 90 Kg/h, preferably 60 Kg/h. Afterward the concentrated mixture is reduced to powder with a spray-drier. The material obtained has a protein content of 85%. It seems that some starch is in any case dissolved during extraction at 50°C.

### EXAMPLE 2: gluten-free bread

A recipe for gluten-free bread was developed using the proteins extracted from white maize flour. The quantities used are shown in Tables 1 and 2. The flour mix in Table 2 is described in Table 1.

**Table 1: flour mix**

| Ingredients | Percentages (weight/weight) |
|---|---|
| Zein and glutelin from white maize flour extracted as per the present invention | 20 |
| Wheat starch | 60 |
| Rice flour | 20 |
| Total | 100 |

**Table 2**

| Ingredients | Percentage on the flour mix (on dry base of dough) (%) |
|---|---|
| Flour mix (made according to Table 1) | |
| Glucose oxydase (Gluzyme Mono 10000 BG, Novozymes. Denmark) | 0.03 |
| Yeast | 5 |
| Sunflower seed oil | 6 |
| Dextrose | 2 |
| Salt | 1 |
| Water | 90 |

All the dry ingredients are mixed at 40°C until a homogeneous mix is obtained in a food-mixer with a heating jacket. Water at 40°C is added to the mix and everything is mixed for 1-3 minutes until a film is formed, similar to that formed by gluten. Then the oil is added and mixing is continued for 8 minutes. The dough is made to rise in a 400g mold for 30 minutes at a temperature of 40°C with 80% humidity. The dough is able to retain gas and to expand until the moment cooking is started. When the dough reaches the edge of the mold it is cooked at 180°C for 25 minutes. The result (fig. 4) is a bread with a specific volume of 4.56 cc/g and has a texture similar to a bread made with wheat flour. After one day it has a hardness, measured on the Kieffer Texture Analyzer, of 324 +/- 30 g. The right balance of the ingredients has developed a gluten-free bread with a specific volume and texture properties comparable to products with gluten (fig. 4).

Table 3 shows the values of softness after 1 day and after 7 days measured on the Texture Analyzer and the specific volume of wheat bread and gluten-free bread according to the recipe in example 2 of the present invention.

**Table 3**

| | Hardness 1 day (g) | Hardness 7 days (g) | S. Volume (cm³/g) |
|---|---|---|---|
| Wheat bread | 270 | 500 | 03.09.00 |
| Gluten-free bread according to example 2 | 324 ± 30 | 773 ± 119 | 04.56.00 |

### EXAMPLE 3: gluten-free pasta

A recipe for gluten-free pasta was developed using the protein extracted from white maize flour. The quantities used are shown in Table 4.

**Table 4: Pasta mix**

| Ingredients | Percentages (weight/weight) |
|---|---|
| Zein and glutelin from white m the present invention aize flour extracted as per | 15 |
| Wheat starch | 65 |
| Rice flour | 20 |
| Total | 100 |

Advantageously, all the dry ingredients are mixed with about 0.3% of the enzyme transglutaminase, until a homogeneous dough is obtained in a food-mixer. The humidity of the dough is preferably around about 30%. The gluten-free pasta was extruded using the following temperature gradient: 50-60-50-20°C. The pasta was dried in a drier at 40°C and at about 70% humidity for 8 hours. With this recipe it is possible to make various types of pasta such as quills, macaroni or spaghetti.

After a sensory analysis performed by a panel of trained tasters, three parameters of the structure of the cooked pasta were evaluated, with a scale of intensity from 0 (parameter not recognized) to 5 (very intense parameter) following the DIN 10969 regulations. Table 5 shows the results obtained from the sensory analysis where one pasta with wheat was compared with one without gluten according to the example in Table 4.

**Table 5**

| | Consistency in chewing | Flouriness | Rubberiness |
|---|---|---|---|
| Wheat pasta | 4 | 0 | 0 |
| Gluten-free pasta according to example 4 | 3 | 1.5 | 2 |

## Claims

1. Method for the production of maize proteins, **characterized in that** it comprises a step of extracting protein fractions comprising zeins and glutelins from white maize and/or its sub-derivatives, such as for example CGM (Corn Gluten Meal), using an aqueous solution of alcohol or alcohol mixes, in the absence of a reducing and purification agent.

2. Method as in claim 1, **characterized in that** it provides to extract one or more of the protein fractions of zeins and glutelins and of other protein fractions not reduced.

3. Method as in claim 1 or 2, **characterized in that** the extraction pH is in a range comprised between about 5 and about 8.

4. Method as in claim 3, **characterized in that** the extraction step also provides to use a buffer to control the pH.

5. Method as in any claim hereinbefore, **characterized in that** the alcohol used is isopropanol.

6. Method as in claim 5, **characterized in that** the concentration of the aqueous solution of isopropanol used is from about 40% to about 90%.

7. Method as in any claim hereinbefore, **characterized in that** the extraction step comprises mixing white maize and/or its sub-derivatives, such as for example CGM (Corn Gluten Meal), with the aqueous solution of alcohol, heating and stirring.

8. Method as in claim 7, **characterized in that** the heating occurs at a temperature from about 35°C to about 60°C.

9. Method as in claim 7 or 8, **characterized in that** the stirring lasts from about 10 minutes to about 100 minutes.

10. Method as in any claim hereinbefore, **characterized in that** the extraction step is repeated a plurality of times.

11. Maize proteins obtainable by means of a method as in any claim hereinbefore.

12. Formula for the preparation of a gluten-free food comprising the maize proteins extracted by means of a method as in any claim hereinbefore.

13. Formula as in claim 12, for the preparation of a gluten-free bakery product, in which the percentage of said maize proteins to be inserted is comprised between about 5% and about 30% of the weight of the dry ingredients of the dough.

14. Formula as in claim 12 or 13, for the preparation of a gluten-free bakery product, in which the percentage of the protein fractions of a higher molecular weight, between 80-160 Kda, such as the glutelins, varies from 2 to 100% of the total of proteins used.

15. Formula as in claim 12, 13 or 14 for the preparation of a gluten-free bakery product, which comprises a glucose oxydase or transglutaminase enzyme added to the starch and/or flour and used in combination with the maize proteins.

16. Method for the preparation of a dough for an bakery product comprising a formula as in any claim from 12 to 15, which provides to maintain the temperature of the dough, both in the processing step of kneading and in the subsequent steps and the leavening, up to the cooking step, at a temperature comprised between about 35°C and about 60°C.

17. Method as in claim 16, in which the processing time of the dough lasts from about 2 minutes to about 90 minutes.

18. Bakery product obtainable by means of a method as in claim 16 or 17.

19. Formula as in claim 12, for the preparation of a gluten-free pasta which includes:
- maize flour and/or rice flour in a percentage from about 10% to about 90% of the weight of the hydrated dough;
- said maize proteins from about 3% to about 7% of the weight of the hydrated dough.

20. Pasta comprising a formula as in claim 19.

## Patentansprüche

1. Verfahren zur Herstelluug von Maisproteinen, **dadurch gekennzeichnet, dass** es einen Schritt umfasst, in dem Proteinfraktionen, die Zeine und Gluteline aus weißem Mais und/oder ihre Unterderivate umfassen, wie zum Beispiel Maisglutenmehl, unter Verwendung einer wässrigen Lösung aus Alkohol oder Alkoholgemischen in Abwesenheit eines Reduktion- und Reinigungsmittels, extrahiert werden.

2. Verfahren nach Anspruch 1, **dadurch gekennzeichnet, dass** ein oder mehrere Proteinfraktionen der Zeine und Gluteline und anderer, nicht-reduzierter Proteinfraktionen extrahiert werden.

3. Verfahren nach Anspruch 1 oder 2, **dadurch gekennzeichnet, dass** der Extraktions pH-Wert in einem Bereich liegt, umfasst zwischen etwa 5 und etwa 8.

4. Verfahren nach Anspruch 3, **dadurch gekennzeichnet, dass** der Extraktionsschritt die Verwendung eines Puffers zur Kontrolle des pH-Wertes bereitstellt.

5. Verfahren nach einer der vorhergehenden Ansprüche, **dadurch gekennzeichnet, dass** der verwendete Alkohol Isopropanol ist.

6. Verfahren nach Anspruch 5, **dadurch gekennzeichnet, dass** die Konzentration der verwendeten, wässrigen Isopropanol-Lösung von etwa 40% bis etwa 90% ist.

7. Verfahren nach einem der vorhergehenden Ansprüche, **dadurch gekennzeichnet, dass** der Extraktionsschritt das Mischen des weißen Maises und/oder seiner Unterderivate, wie zum Beispiel Maisglutenmehl, mit der wässrigen Alkohollösung, Erhitzen und Rühren umfasst.

8. Verfahren nach Anspruch 7, **dadurch gekennzeichnet, dass** das Erhitzen bei einer Temperatur von etwa 35°C bis etwa 60 °C erfolgt.

9. Verfahren nach Anspruch 7 oder 8, **dadurch gekennzeichnet, dass** das Rühren etwa 10 Minuten bis etwa 100 Minuten andauert.

10. Verfahren nach einer der vorhergehenden Ansprüche, **dadurch gekennzeichnet, dass** der Extraktionsschritt mehrere Male wiederholt wird.

11. Maisproteine erhältlich nach einem Verfahren der vorhergehenden Ansprüche.

12. Rezept für die Herstellung eines Gluten-freien Lebeusmittels, umfassend die Maisproteine extrahiert nach einem Verfahren der vorhergehenden Ansprüche.

13. Rezept nach Anspruch 12 für die Herstellung einer Gluten-freien Backware, in welcher der prozentuale Anteil des eingesetzten Maisprodukts zwischen etwa 5 % und etwa 30 % des Gewichts der trockenen Bestandteile des Teigs ist.

14. Rezept nach Anspruch 12 oder 13 für die Herstellung einer Gluten-freien Backware, in welcher der prozentuale Anteil der Proteinfraktionen mit einem höheren Molekulargewicht zwischen 80-160 KDa, wie zum Beispiel Gluteline, zwischen 2 bis 100 % des gesamten verwendeten Proteins variiert.

15. Rezept nach Anspruch 12, 13 oder 14, zur Herstellung einer Gluten-freien Backware, welche ein Glucose-Oxydase- oder ein Transglutaminase-Enzym umfasst, das zu der Stärke und/oder zu dem Mehl hinzugegeben und in Kombination mit den Maisproteinen verwendet wird.

16. Verfahren zur Herstellung eines Teigs für eine Backware, umfassend ein Rezept nach einem der Ansprüche 12 bis 15, welches die Temperatur des Teiges, sowohl im Knetschritt als auch in den darauffolgenden Schritten, und während des Gehenlassens bis zum Backschritt bei einer Temperatur zwischen etwa 35 °C und etwa 60 °C aufrechterhält.

17. Verfahren nach Anspruch 16, in welchem die Bearbeitungszeit des Teigs von etwa 2 Minuten bis etwa 90 Minuten andauert.

18. Backware erhältlich nach einem der Ansprüche 16 oder 17.

19. Rezept nach Anspruch 12 für die Herstellung einer Gluten-freien Pasta, welche beinhaltet:
- Maismehl und/ oder Reismehl in einem prozentualen Anteil von etwa 10
% bis etwa 90 % des Gewichtes des hydratisierten Teigs;
- Das Maisprotein mit von etwa 3 % bis etwa 7 % des Gewichts des hydratisierten Teigs.

20. Pasta umfassend ein Rezept nach Anspruch 19.

## Revendications

1. Procédé pour la production de protéines de maïs, **caractérisé en ce qu'**il comprend une étape d'extraction des fractions protéiques et comprenant les zéines et les glutélines du maïs blanc et / ou ses sous-produits dérivés, tels que par exemple la FGM (farine de gluten de maïs), en utilisant une solution aqueuse d' l'alcool ou de mélanges d'alcool, en l'absence d'un agent réducteur et de purification.

2. Procédé selon la revendication 1, **caractérisé en ce qu'**il prévoit d'extraire une ou plusieurs des fractions protéiques de zéines et de glutélines et d'autres fractions protéiques non réduites.

3. Procédé selon la revendication 1 ou 2, **caractérisé en ce que** le pH d'extraction est dans une plage comprise entre environ 5 et environ 8.

4. Procédé selon la revendication 3, **caractérisé en ce que** l'étape d'extraction prévoit également l'utilisation d'un tampon pour contrôler le pH.

5. Procédé selon l'une quelconque des revendications précédentes, **caractérisé en ce que** l'alcool utilisé est l'isopropanol.

6. Procédé selon la revendication 5, **caractérisé en ce que** la concentration de la solution aqueuse d'isopropanol utilisée est d'environ 40% à environ 90%.

7. Procédé selon l'une quelconque des revendications précédentes, **caractérisé en ce que** l'étape d'extraction comprend le mélange de maïs blanc et / ou de ses sous-produits dérivés, tels que par exemple FGM (farine de gluten de maïs), avec la solution aqueuse d'alcool, en chauffant et en agitant.

8. Procédé selon la revendication 7, **caractérisé en ce que** le chauffage s'effectue à une température d'environ 35°C à environ 60°C.

9. Procédé selon la revendication 7 ou 8, **caractérisé en ce que** l'agitation dure d'environ 10 minutes à environ 100 minutes.

10. Procédé selon l'une quelconque des revendications précédentes, **caractérisé en ce que** l'étape d'extraction est répétée une pluralité de fois.

11. Protéines de maïs susceptibles d'être obtenu au moyen d'un procédé selon l'une quelconque des revendications précédentes.

12. Formule pour la préparation d'un aliment sans gluten, comprenant les protéines de maïs extraites au moyen du procédé selon l'une quelconque des revendications précédentes.

13. Formule selon la revendication 12, pour la préparation d'un produit de boulangerie sans gluten, dans lequel le pourcentage desdites protéines de maïs à ajouter est compris entre environ 5% et environ 30% du poids des ingrédients secs de la pâte.

14. Formule selon la revendication 12 ou 13, pour la préparation d'un produit boulangerie sans gluten, dans laquelle le pourcentage des fractions protéiques de poids moléculaire plus élevé, entre 80 et160 kDa, tels que les glutélines, varie de 2 à 100% du total des protéines utilisées.

15. Formule selon la revendication 12, 13 ou 14 pour la préparation d'un produit boulangerie sans gluten, qui comprend une glucose oxydase ou une enzyme transglutaminase ajoutée à l'amidon et / ou la farine et utilisé en combinaison avec les protéines de maïs.

16. Procédé pour la préparation d'une pâte pour un produit de boulangerie comprenant une formule selon l'une quelconque des revendications 12 à 15, qui prévoit de maintenir la température de la pâte, à la fois dans l'étape de traitement de malaxage et au cours des étapes ultérieures et le levage, jusqu'à à l'étape de cuisson, à une température comprise entre environ 35°C et environ 60°C.

17. Procédé selon la revendication 16, dans lequel le temps de traitement de la pâte dure d'environ 2 minutes à environ 90 minutes.

18. Produit de boulangerie susceptible d'être obtenu au moyen d'un procédé selon la revendication 16 ou 17.

19. Formule selon la revendication 12, pour la préparation de pâtes sans gluten comprenant :
- de la farine de maïs et / ou de la farine de riz dans une proportion d'environ 10% à environ 90% du poids de la pâte hydratée,
- ledit protéines de maïs d'environ 3% à environ 7% du poids de la pâte hydratée.

20. Påtes comprenant une formule selon la revendication 19.
